# EUROPÄISCHE PATENTSCHRIFT

(11) **EP 0 620 749 B1**
(45) Veröffentlichungstag und Bekanntmachung des Hinweises auf die Patenterteilung: **09.08.1995**
(21) Anmeldenummer: 93901728.1
(22) Anmeldetag: 30.12.1992
(51) Int. Cl.: A61M 25/06

(54) **SPALTKANÜLE UND VERFAHREN ZUR HERSTELLUNG EINER SOLCHEN**
PRE-SLIT CANNULA NEEDLE AND METHOD OF PRODUCING IT
CANULE CLIVABLE ET SON PROCEDE DE FABRICATION

(30) Priorität: 08.01.1992 DE 4200255; 13.05.1992 US 882533
(43) Veröffentlichungstag der Anmeldung: 26.10.1994
(73) Patentinhaber: SÜDDEUTSCHE FEINMECHANIK GMBH, D-63607 Wächtersbach (DE)
(72) Erfinder: HIRSCH, Cristian, D-6454 Bruchköbel (DE); SCHLEGEL, Karlheinz, D-6456 Langenselbold (DE)
(74) Vertreter: Stoffregen, Hans-Herbert, Dr. Dipl.-Phys.
(86) Internationale Anmeldenummer: EP9203010
(87) Internationale Veröffentlichungsnummer: WO9313821

(56) Entgegenhaltungen:
- EP-A- 0 064 212
- EP-A- 0 245 837
- EP-A- 0 435 157
- WO-A-82/03558
- WO-A-83/03766
- GB-A- 1 381 053
- US-A- 4 776 846
- US-A- 4 888 000

## Beschreibung

Die Erfindung bezieht sich auf eine aus Metall bestehende Spaltkanüle mit zumindest einer in Längsrichtung verlaufender Sollbruchlinie, vorzugsweise jedoch auf eine Spaltkanüle mit zwei in Längsrichtung verlaufenden Sollbruchlinien. Ferner bezieht sich die Erfindung auf ein Verfahren zur Herstellung von zumindest einer in Längsrichtung einer Spaltkanüle verlaufenden Sollbruchlinie durch Bearbeitung eines ablängbaren bzw. abgelängten Rohres.

Um z. B. Katheter einzuführen, werden in der Medizin sogenannte Spaltkanülen benötigt, die in Richtung der Kanülenlängsachsen Sollbruchlinien aufweisen (DE-C 2 104 211). Durch diese Sollbruchlinien soll die Möglichkeit gegeben werden, nach Einführen der Kanüle und des durch diese einführbaren Katheters nach Herausziehen der Kanüle diese von Hand in zwei Hälften zu zerlegen. Dabei soll mit möglichst wenig Kraft ein Zerlegen möglich sein, wobei gleichzeitig sicherzustellen ist, daß ein sogenanntes "Aufreißen" über die ganze Länge erfolgt, daß also nicht ein Teil der Kanüle abbricht. Dies kann jedoch dann erfolgen, wenn die Wandstärken innerhalb der Sollbruchlinien keine definierten Restwandstärken aufweisen. Letzteres kann auch dazu führen, daß eine Kanülenhälfte abknickt, so daß die weitere Handhabung überaus problematisch werden kann.

Die Sollbruchlinien der bekannten Spaltkanülen werden spanabhebend z. B. durch Fräsen hergestellt. Dabei wird vom Nutzer wegen bestehender Infektionsgefahren wie AIDS unter anderem vorgeschrieben, daß die Kanülen frei von scharfen Kanten oder Graten sind.

Erwähntermaßen soll feiner die Restwandstärke innerhalb der Sollbruchlinie, die nur einige Hundertstelmillimeter betragen kann, gleichmäßig über die Länge der Kanüle sein. Insbesondere ist erforderlich, daß die Restwandstärken der gegenüberliegenden Sollbruchlinien übereinstimmen, um ein gleichmäßiges und müheloses Aufreißen zu erlauben.

Um all diese Bedingungen zu erfüllen, sind aufwendige Herstellungsverfahren und regelmäßige Zerstörungsprüfungen an den fertigen Spaltkanülen erforderlich. Da ferner Fertigung und Handling von Hand erfolgt, sind hohe Produktionskosten gegeben.

Ferner sind Spaltkanülen aus Kunststoffmaterial bekannt, die durch Extrudieren hergestellt werden. Dabei wird gleichzeitig eine Sollbruchlinie ausgeformt (US 4 776 846, EP 0 245 837 A2)
Nach der EP 0 435 157 A1 kann in einer Spaltkanüle durch Ultraschall eine Sollbruchlinie ausgebildet werden.

Der vorliegenden Erfindung liegt das Problem zugrunde eine Spaltkanüle bzw. ein Verfahren zu deren Herstellung derart zur Verfügung zu stellen, daß eine definierte Restwandstärke über die gesamte Länge der Sollbruchlinie gegeben ist, wobei Abweichungen der Restwandstärken in den einzelnen Sollbruchlinien nicht gegeben sein sollen. Auch sollen Kanten, Grate oder andere zu Verletzungen führende Unebenheiten an der Spaltkanülenaußenwandung ausgeschlossen sein. Ferner sollen bei geringem herstellungstechnischem Aufwand reproduzierbare Ergebnisse erzielbar sind.

Das Problem wird erfindungsgemäß bei einer aus Metall bestehenden Spaltkanüle der eingangs beschriebenen Art dadurch gelöst, daß die Sollbruchlinie durch spanloses Umformen hergestellt ist.

Erfindungsgemäß erfolgt nicht durch Fräsen oder andere spanabhebende Bearbeitungen das Ausbilden der vorzugsweise zwei Sollbruchlinien, wodurch einerseits aufgrund der erforderlichen Überprüfungen und gegebenenfalls Nachbearbeitungen hohe Produktionskosten entstehen und andererseits nicht ausgeschlossen werden kann, daß die Restwand` stärken innerhalb einer Sollbruchlinie oder von Sollbruchlinie zu Sollbruchlinie in einer Spaltkanüle variieren. Vielmehrerfolgt erfindungsgemäß durch spanloses Umformen eine Materialverdrängung, die definierte Restwandstärken sicherstellt. Hierdurch ergibt sich der Vorteil, daß nur stichprobenartige Überprüfungen der Spaltkanülen erfolgen müssen. Eine Überprüfung der Außenwandungen selbst ist gleichfalls nur von Fall zu Fall erforderlich, da durch die spanlose Umformung eine Aufrauhung oder Ausbildung von scharfen Kanten an der Außenfläche der fertigen bzw. weitgehend fertigen Kanüle nicht möglich ist.

Da die erfindungsgemäße Spaltkanüle keine scharfen Kanten oder Grate aufweist, ist die Gefahr von Verletzungen ausgeschlossen.

Durch das spanlose Bearbeiten ist des weiteren der Vorteil gegeben, daß auch Kanülen mit sehr kleinem Durchmesser als Spaltkanülen ausgebildet werden können. Durchmesser kleiner als 1 mm wie z. B. 0,7 mm können erfindungsgemäß ausgebildet werden. Auch ist eine Längenunabhängigkeit gegeben.

In Ausgestaltung kann die Spaltkanüle innerhalb der Sollbruchlinien perforiert sein,so daß ein noch einfacheres Zerlegen der Kanüle möglich ist.

Ein Verfahren zur Herstellung einer Spaltkanüle zeichnet sich dadurch aus, daß die Sollbruchlinie durch spanloses Formgebungsverfahren hergestellt wird. Dabei kann in Längsrichtung des Rohres auf dessen Außenwandung ein Prägewerkzeug wie Kerbstempel einwirken, dem ein innerhalb des Rohres verlaufender Innenstempel zugeordnet ist. Auf diese Weise erfolgt eine definierte Materialverdrängung beim spanlosen Umformen, so daß geringe Herstellungstoleranzen vorliegen. Insbesondere erfolgt das Einprägen bei einer fertigen Kanüle, die also geschliffen und gegebenenfalls in erforderlichem Umfang behandelt worden ist.

Durch das erfindungsgemäße Verfahren erfolgt eine wesentliche Verringerung von nötigen Fertigungsschritten und somit eine Verbesserung der Wirtschaftlichkeit. Kanten, Zähne oder Späne entlang der Sollbruchlinie entfallen vollständig.

Erwähntermaßen kann die Gleichmäßigkeit der Sollbruchlinien, also die innerhalb dieser verlaufenden Restwandstärken erhöht und somit die Kraft zum Zerlegen der Kanüle verringert werden. Hierdurch ergibt sich eine einfache und sichere Handhabung der Spaltkanüle im medizinischen Einsatz.

In Ausgestaltung der Erfindung kann das Prägewerkzeug wie Kerbstempel ein über die Länge der Kanüle sich erstreckendes schneid- oder schwertartiges Werkzeug sein, dessen der Kanüle zugewandte und die Formgebung hervorrufende Schneide eine derartige Geometrie aufweist, daß zwei äußere gewölbte Abschnitte jeweils über eine Vertiefung wie Einbuchtung in einen V-förmigen Vorsprung übergehen, durch dessen Höhe die Einprägetiefe in der Kanülenwandung bestimmt wird.

Vorzugsweise werden zwei diametral gegenüberliegenden Sollbruchlinien durch Drehen des Rohres um 180° eingeformt, so daß nur ein Kerbstempel oder ein gleichwirkendes Element benötigt wird.

Alternativ besteht selbstverständlich die Möglichkeit, daß auch auf diametral gegenüberliegenden Linien gleichzeitig oder nacheinander jeweils ein gesondertes Werkzeug einwirkt.

Auch besteht die Möglichkeit, die Sollbruchlinien in bezug auf die Längsachse der Kanüle entlang Strahlen verlaufen zu lassen, die einen spitzen Winkel einschließen.

Weitere Einzelheiten, Vorteile und Merkmale der Erfindung ergeben sich nicht aus den Ansprüchen, den diesen zu entnehmenden Merkmalen - für sich und/oder in Kombination -, sondern auch aus der nachfolgenden Beschreibung eines der Zeichnung zu entnehmenden bevorzugten Ausführungsbeispiels, durch das das erfindungsgemäße Verfahren zur Herstellung von Spaltkanülen verdeutlicht werden soll.

Es zeigen:
- Fig. 1: eine Seitenansicht einer Spaltkanüle,
- Fig. 2: eine Draufsicht einer Spaltkanüle,
- Fig. 3: einen Schnitt entlang der Linie A-B in Fig. 2, jedoch in vergrößerter Darstellung,
- Fig. 4: eine teilweise aufgerissene Spaltkanüle,
- Fig. 5: einen Ausschnitt aus einem Prägewerkzeug und
- Fig. 6: eine Prinzipdarstellung zur Verdeutlichung eines Verfahrens zur Ausbildung von Sollbruchlinien in eine Kanüle.

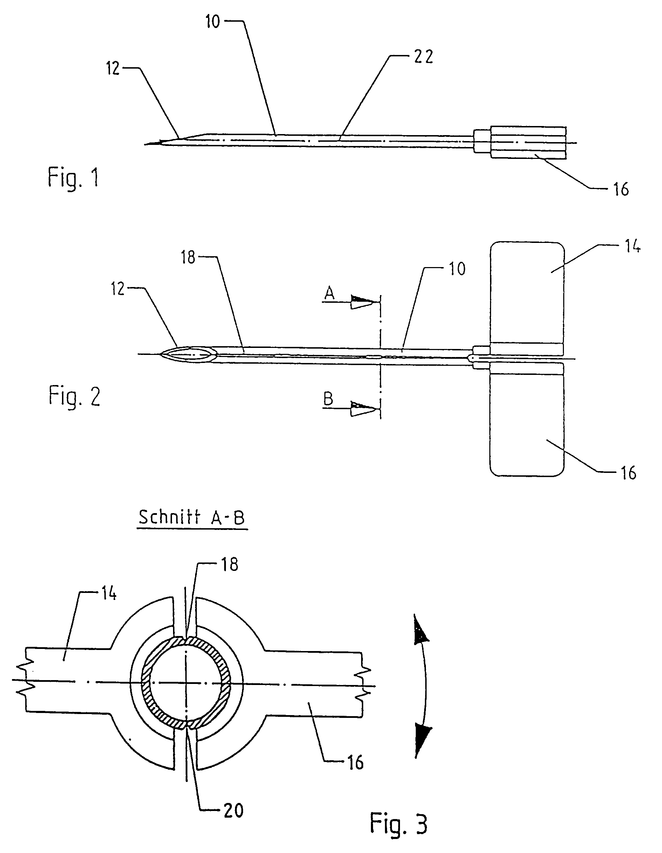

In Fig. 1 ist eine Seitenansicht einer aus Metall wie Edelstahl bestehenden Spaltkanüle (10) mit schräg angeschliffener Spitze (12) und im Endbereich vorzugsweise umspritzten Flügeln (14) und (16) dargestellt, die ein Handhaben zum Aufreißen der Spaltkanüle entlang von Sollbruchlinien (18) und (20) ermöglicht.

Sind in den Figuren die Sollbruchlinien diametral in bezug auf die Längsachse (22) der Kanüle (10) dargestellt, so können diese auch in anderen Bereichen in die Wandung der Kanüle (10) eingeprägt sein.

Durch die Sollbruchlinien (18) und (20) wird die Möglichkeit geschaffen, die Kanüle durch Auseinanderbewegen der Flügel (14) und (16) auseinanderzureißen, um auf diese Weise die Kanüle (10) von einem zuvor durch diese geschobenen Katheter zu entfernen.

Um die Sollbruchlinien (18) und (20) in der Wandung der Kanüle (10) durch spanloses Umformen auszubilden, wird verfahrensmäßig folgendes durchgeführt.

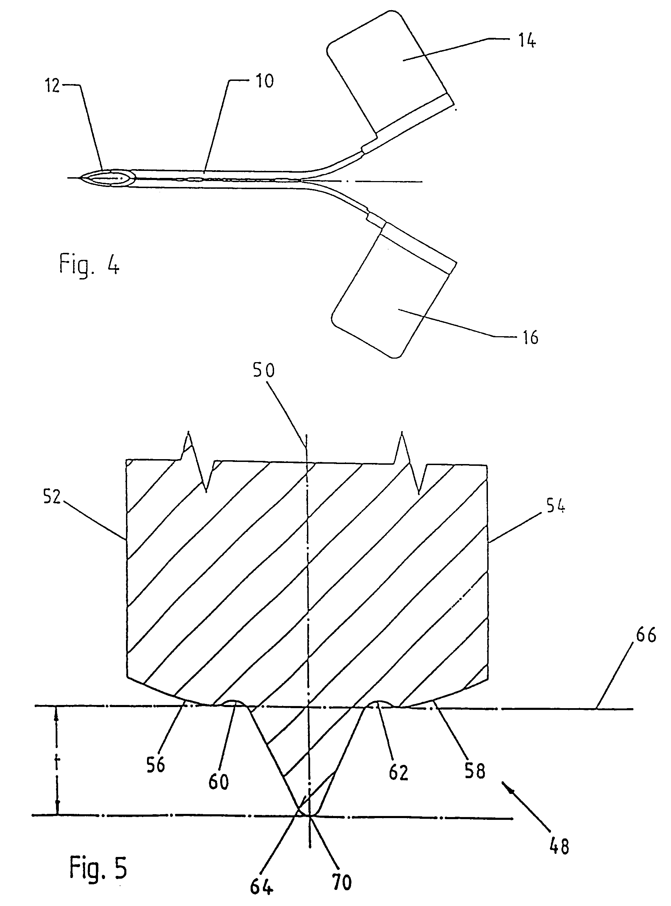

In Fig. 5 ist im Querschnitt ein Schneidkantenbereich eines Werkzeuges dargestellt, mit dem die Sollbruchlinien (18) und (20) durch spanloses Umformen und in bezug auf eine Sollbruchlinie durch einen einzigen Prägegang ausgebildet werden sollen, wobei eine automatische Tiefenregulierung der Sollbruchlinie erfolgt.

Hierzu weist die Prägekante (48) des sich über die Länge der Kanüle (10) erstreckenden Werkzeugs, das als Schwert zu bezeichnen ist, prinzipiell die der Fig. 5 zu entnehmenden Form auf. Das heißt, daß in bezug auf eine Symmetrieebene (50) von Seitenflächen (52) und (54) des Werkzeuges konvex ausgebildete Erhebungen (56) und (58) ausgehen, die über in Längsrichtung verlaufende Vertiefungen wie Einkerbungen (60) und (62) in eine im Schnitt V-förmige Erhebung (64) übergehen. Dabei gibt der Abstand zwischen den Kuppen der seitlichen Erhebungen (56) und (58) und der Spitze des Vorsprunges (64) die Tiefe der Sollbruchlinien (18) bzw. (20) vor.

Um die Spaltkanüle (10) mit diametral gegenüberliegenden Sollbruchlinien (18) und (20) herzustellen, wird die fertige Kanüle (10) zwischen Anlagen (22), (24), (26) und (28) positioniert. Innerhalb der Kanüle (10) wird ein Gegenstempel (30) - auch Füllstab genannt - eingebracht.

Um in die Wandung (32) der Kanüle (10) die Sollbruchlinien (18) bzw. (20) spanlos einzuprägen, sind in Längsrichtung der Kanüle (10) verlaufende und konisch zur Spitze hin verlaufende Präge- oder Kerbstempel (34) bzw. (36) vorgesehen, die zwischen den Anlagen bzw. Halterungen (22) und (26) bzw. (24) und (28) auf die Rohrwandung (32) einwirken. Die Prägestempel (34) bzw. (36) können schneidkantenseitig eine Geometrie aufweisen, die der Prägekante (48) entspricht.

Durch die Krafteinwirkung auf die Prägestempel (34) bzw. (36) in Richtung der Längsachse (12), also des Mittelpunktes der Kanüle (10) erfolgt eine Materialverdrängung, wobei durch nicht dargestellte Anschläge das Eindringen des Prägestempels (34) bzw. (36) definiert vorgegeben werden kann. Hierdurch ist gewährleistet, daß die Restwandstärke (38) bzw. (40), also der Abstand zwischen Rohrinnenwandung (42) und tiefstem Punkt (44) bzw. (46) der Sollbruchlinie (18) bzw. (20) fest vorgebbar ist.

Wird ein Werkzeug mit der Prägekante (48) verwendet, so stellt sich eine Einprägetiefe automatisch ein.

Es ergeben sich durch das erfindungsgemäße Verfahren folglich definierte Restwandstärken (38) und (40), die gewährleisten. daß ein problemloses Aufreißen der Spaltkanüle (10) möglich wird.

Da das Ausbilden der Sollbruchlinien (18) und (20) durch spanloses Umformen erfolgt, bilden sich an der Außenfläche der Kanüle (10) keine Unebenheiten wir Kanten, Grate oder ähnliches, so daß weiterhin eine glatte Oberfläche gegeben ist. Die Gefahr von Verletzungen ist damit ausgeschlossen. Auch ist eine Nachbearbeitung der Außenwandung nicht notwendig. Folglich kann die Kanüle (10) vor deren spanloser Bearbeitung fertig bearbeitet sein.

Durch das spanlose Umformen erfolgt eine Materialverstärkung unmittelbar neben den Sollbruchlinien (18) und (20), die in der Zeichnung deutlich erkennbar ist.

Sind im Ausführungsbeispiel in bezug auf die Längsachse (12) zwei diametral zueinander angeordnete Prägewerkzeuge (34) und (36) vorgesehen, so kann das Ausbilden der Sollbruchlinien (18), (20) auch nur durch einen einzigen Stempel (34), erfolgen. In diesem Fall muß nach Ausbilden der Sollbruchlinie (18) die Kanüle (10) um einen gewünschten Winkel α, vorzugsweise 180° gedreht werden, damit die Kanüle (10) unterhalb des Prägestempels (34) in der Position zu liegen kommt, in der die Sollbruchlinie (20) verlaufen soll.

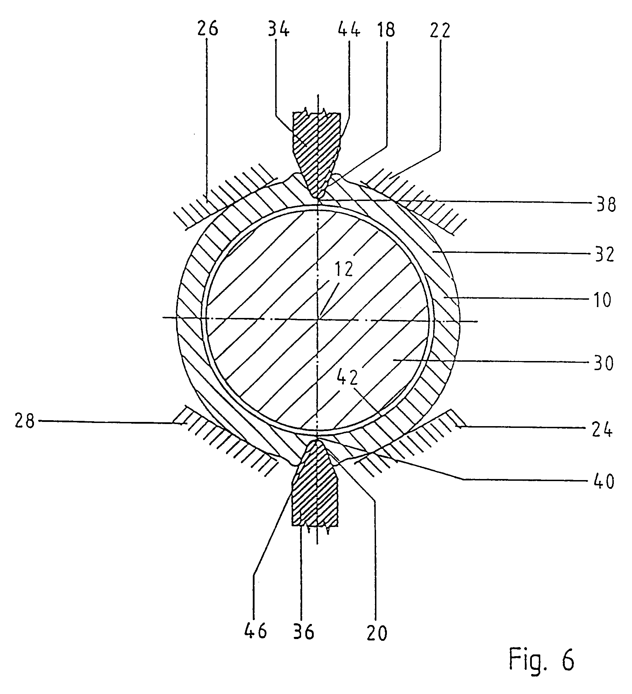

Wie die Fig. 6 verdeutlicht, sind die Prägewerkzeuge (34) und (36) im Schnitt keilförmig ausgebildet. Dabei verläuft die entlang der Kanülenachse (12) verlaufende Kante parallel zu dieser.

Alternativ besteht die Möglichkeit daß die Kante des Werkzeugs in Längsrichtung gewellt ist, um auf diese Weise eine derartige Restwandstärkenvariation innerhalb der Sollbruchlinien (18) und (20) hervorzurufen, daß sich eine Perforation ausbildet.

Wie die Fig. 5 verdeutlicht, gibt der Abstand zwischen einer die Erhebungen (56) und (58) tangential berührenden Linie (66) und der Spitze (70) des Vorsprunges (64) die Tiefe t der in die Kanülenwandung (32) einzuprägenden Sollbruchlinie (18) bzw. (20) vor.

Die Wandstärke der Kanüle (10) kann zwischen 0,08 und 0,2 mm liegen. Die Restwandstärke in den Sollbruchlinien liegt vorzugsweise zwischen 30 und 50 »m. Die maximale Stärke im Bereich des Aufwurfs, also der Erhebungen (56) und (58) beträgt sodann 15 bis 25 »m.

## Patentansprüche

1. Spaltkanüle (10) bestehend aus Metall mit zumindest einer in Längsrichtung verlaufenden Sollbruchlinie (18, 20),
**dadurch gekennzeichnet,**
daß die Sollbruchlinie (18, 20) durch spanloses Umformen hergestellt ist.

2. Spaltkanüle nach Anspruch 1,
**dadurch gekennzeichnet,**
daß die Sollbruchlinien (18, 20) perforiert sind.

3. Spaltkanüle nach Anspruch 1,
**dadurch gekennzeichnet,**
daß die Spaltkanüle (10) im Bereich unmittelbar neben der Sollbruchlinie (18, 20) eine stärkere Wanddicke als die Wanddicke der Kanüle selbst aufweist.

4. Spaltkanüle (10) bestehend aus Metall mit umlaufend geschlossenem Kanülenkörper, angeschliffener Kanülenspitze (12) und im der Kanülenspitze abgewandten Endbereich vorhandener Handhabe (14, 16) zum zumindest teilweise Auftrennen des Kanülenkörpers entlang von zwei in Längsrichtung des Kanülenkörpers verlaufenden Sollbruchlinien (18, 20), **dadurch gekennzeichnet**, daß die Sollbruchlinien (18, 20) durch spanloses Umformen in dem Kanülenkörper ausgebildet sind.

5. Verfahren zur Herstellung von zumindest einer in Längsrichtung einer aus Metall bestehenden Spaltkanüle (10) verlaufenden Sollbruchlinie (18, 20) durch Bearbeiten eines Kanülenkörpers,
**dadurch gekennzeichnet,**
daß die Sollbruchlinie (18, 20) durch spanloses Formgebungsverfahren hergestellt wird.

6. Verfahren nach Anspruch 5,
**dadurch gekennzeichnet,**
daß in Längsrichtung des Kanülenkörpers auf dessen Außenwandung (32) ein Prägewerkzeug (34, 36) einwirkt, dem ein innerhalb des Rohres verlaufendes Gegenwerkzeug (30) zugeordnet wird.

7. Verfahren nach Anspruch 5,
**dadurch gekennzeichnet,**
daß zwei Sollbruchlinien (18, 20) nacheinander durch Drehen der Kanüle (10) um einen Winkel α in die Rohrwandung (32) eingeprägt werden.

8. Verfahren nach Anspruch 5,
**dadurch gekennzeichnet,**
daß zwei Sollbruchlinien (18, 20) gleichzeitig in den umfangsseitig geschlossenen Kanülenkörper eingeprägt werden.

9. Verfahren nach Anspruch 5,
**dadurch gekennzeichnet,**
daß sich in Längsrichtung des aus Metall bestehenden Kanülenkörpers und über dessen Länge ein Werkzeug (34, 36) mit einer Prägekante zum Ausbilden der Sollbruchlinie (18, 20) erstreckt, daß die Prägekante (48) zu einer Symmetrieebene, in der die Längsachse (12) verläuft, seitliche in Richtung des Kanülenkörpers verlaufende Erhebungen (56, 58) aufweist, die in einem im Schnitt V-förmigen Vorsprung (64) mit einer Spitze (70) übergehen, der symmetrisch zur Symmetrieebene ausgebildet ist, wobei der Abstand zwischen einer die Erhebung tangential berührenden Linie (66) und der Spitze (70) in etwa die Tiefe (t) der Sollbruchlinie bestimmt.

## Claims

1. Slit cannula (10) made of metal, having at least one predetermined breaking line (18, 20) extending in a longitudinal direction,
characterized in that
the predetermined breaking line (18, 20) is manufactured by non-cutting shaping.

2. Slit cannula according to claim 1,
characterized in that
the predetermined breaking lines (18, 20) are perforated.

3. Slit cannula according to claim 1,
characterized in that
the slit cannula (10) in the region immediately adjacent to the predetermined breaking line (18, 20) has a greater wall thickness than the wall thickness of the cannula itself.

4. Slit cannula (10) made of metal, having a peripherally closed cannula body, an integrally ground cannula tip (12) and a handle (14, 16) provided in the end region remote from the cannula tip for the purpose of at least partially splitting open the cannula body along two predetermined breaking lines (18, 20) extending in a longitudinal direction of the cannula body, characterized in that the predetermined breaking lines (18, 20) are formed in the cannula body by non-cutting shaping.

5. Method of manufacturing at least one predetermined breaking line (18, 20) extending in a longitudinal direction of a metal slit cannula (10) through working of a cannula body,
characterized in that
the predetermined breaking line (18, 20) is manufactured by a non-cutting shaping process.

6. Method according to claim 5,
characterized in that
acting in a longitudinal direction of the cannula body upon the outer wall (32) thereof is a stamping tool (34, 36), associated with which is a counter-tool (30) extending inside the tube.

7. Method according to claim 5,
characterized in that
two predetermined breaking lines (18, 20) are impressed successively into the tube wall (32) by rotating the cannula (10) through an angle α.

8. Method according to claim 5,
characterized in that
two predetermined breaking lines (18, 20) are impressed simultaneously into the peripherally closed cannula body.

9. Method according to claim 5,
characterized in that
a tool (34, 36) having a stamping edge for forming the predetermined breaking line (18, 20) extends in a longitudinal direction and over the length of the metal cannula body, that the stamping edge (48) in relation to a plane of symmetry, in which the longitudinal axis (12) extends, has lateral raised portions (56, 58) which extend in the direction of the cannula body and verge into a projection (64), which is V-shaped in cross-section, has a tip (70) and is formed symmetrically to the plane of symmetry, the distance between a line (66) tangentially touching the raised portion and the tip (70) substantially determining the depth (t) of the predetermined breaking line.

## Revendications

1. Canule clivable (10), en métal, présentant au moins une ligne longitudinale continue de rupture imposée (18, 20), caractérisée en ce que la ligne de rupture (18, 20) est obtenue par formage.

2. Canule selon la revendication 1, caractérisée en ce que les lignes de rupture imposée (18, 20) sont perforées.

3. Canule selon la revendication 1, caractérisée en ce qu'elle présente dans la zone bordant directement la ligne de rupture (18, 20), une épaisseur de paroi supérieure à celle du reste de la canule.

4. Canule clivable (10) en métal, comportant un corps de canule à périphérie fermée, une pointe de canule (12) affûtée et, à l'extrémité opposée à cette pointe, un organe de prise (14, 16) permettant de séparer, au moins partiellement, le corps de la canule le long de deux lignes de rupture imposée (18, 20) disposées longitudinalement sur le corps, caractérisée en ce que les lignes de rupture (18, 20) sont réalisées dans le corps de la canule par formage.

5. Procédé d'obtention d'au moins une ligne de rupture imposée (18, 20) longitudinale, sur une canule clivable (10) en métal, par usinage d'un corps de canule, caractérisé en ce que la ligne de rupture (18, 20) est obtenue par un procédé de formage.

6. Procédé selon la revendication 5, caractérisé en ce qu'on fait agir selon la direction longitudinale du corps de la canule, sur sa paroi externe (32), un outil d'estampage (34, 36), auquel est associé un outil de contrepartie (30) occupant l'intérieur du tube.

7. Procédé selon la revendication 5, caractérisé en ce que deux lignes de rupture imposée (18, 20) sont réalisées par empreinte dans la paroi tubulaire (32), l'une après l'autre, en faisant tourner la canule (10) d'un angle α.

8. Procédé selon la revendication 5, caractérisé en ce que deux lignes de rupture (18, 20) sont réalisées simultanément, par empreinte, dans le corps à périphérie fermée de la canule.

9. Procédé selon la revendication 5, caractérisé en ce que :
- un outil (34, 36) portant un poinçon de formage de la ligne de rupture imposée (18, 20) est disposé longitudinalement par rapport au corps métallique de la canule, sur toute la longueur de ce corps ;
- le poinçon de formage (48) est symétrique par rapport à un plan (50) passant par l'axe longitudinal (12) du corps, il présente latéralement des côtes (56., 58) parallèles à la direction du corps et se prolongeant par une partie en saillie (64) à section en V terminée par une pointe (70), symétrique par rapport au plan (50) ;
- la distance entre une ligne (66) tangente aux côtes et la pointe (70) correspond sensiblement à la profondeur (t) de la ligne de rupture imposée.
